# EUROPÄISCHE PATENTSCHRIFT

(11) **EP 1 830 742 B1**
(45) Veröffentlichungstag und Bekanntmachung des Hinweises auf die Patenterteilung: **23.07.2008**
(21) Anmeldenummer: 05826005.0
(22) Anmeldetag: 22.12.2005
(51) Int. Cl.: A61F 2/00

(54) **Prothesenvorrichtung**
Prosthetic device
Système de prothèse

(30) Priorität: 22.12.2004 DE 102004063055
(43) Veröffentlichungstag der Anmeldung: 12.09.2007
(73) Patentinhaber: OPED AG, 6312 Steinhausen (CH)
(72) Erfinder: HASSLER, Andreas, 83101 Rohrdorf (DE)
(74) Vertreter: Tappe, Hartmut
(86) Internationale Anmeldenummer: PCT/EP2005/013944
(87) Internationale Veröffentlichungsnummer: WO 2006/066951

(56) Entgegenhaltungen:
- WO-A-95/31951
- DE-A1- 3 229 812
- US-A- 5 571 206
- US-A1- 2004 158 332
- US-B1- 6 368 357

## Beschreibung

Die vorliegende Erfindung betrifft eine Prothesenvorrichtung mit einer Schalenanordnung zur Aufnahme eines Amputationsstumpfes und einer Rahmeneinrichtung zur Verbindung der Schalenanordnung mit einer Prothese nach Anspruch 1.

Prothesenvorrichtungen der eingangs genannten Art dienen zum Anschluss einer Prothese an einen durch Amputation einer oberen oder unteren Extremität ausgebildeten Amputationsstumpf. Im Falle der Amputation einer unteren Extremität, beispielsweise am Bein unterhalb des Kniegelenks, kann die Prothesenvorrichtung zum sicheren Anschluss einer Prothese, wie beispielsweise einer Gehstütze, an den verbliebenen Unterschenkelstumpf dienen. Im Falle einer Amputation im Unterarmbereich unterhalb des Ellbogengelenkes kann eine derartige Prothesenvorrichtung zum sicheren Anschluss einer die Handfunktionen ersetzenden prothetischen Greifeinrichtung verwendet werden.

Üblicherweise werden derartige Prothesenvorrichtungen, die den mechanischen Anschluss von Prothesen an den jeweiligen Gliederstumpf ermöglichen, erst nach abgeschlossener "Stumpfreifung" an das betroffene Körperteil angeschlossen. Nachfolgend dem operativen Eingriff, mit dem die eigentliche Amputation durchgeführt wird, werden bislang sogenannte "Interimsprothesen" eingesetzt, die so ausgebildet sind, dass sie dem infolge der Wundheilung sich ändernden Umfang des Amputationsstumpfes angepasst werden können. Dabei dienen derartige Interimsprothesen in erster Linie dazu, durch einen dem Umfang des Stumpfes jeweils anpassbaren Umfangsdruck die Stumpfreifung durch eine Förderung des Abschwellvorgangs zu unterstützen.

Ein Beispiel der eingangs genannten Prothesenvorrichtungen ist in DE-A-3 229 812 beschrieben.

Der vorliegenden Erfindung liegt die Aufgabe zu Grunde, eine Prothesenvorrichtung der eingangs genannten Art vorzuschlagen, die sowohl während der Stumpfreifung als auch nach Abschluss der Stumpfreifung zum Anschluss einer Funktionsprothese verwendbar ist.

Diese Aufgabe wird durch eine Prothesenvorrichtung mit den Merkmalen des Anspruchs 1 gelöst.

Bei der erfindungsgemäßen Prothesenvorrichtung mit einer Schalenanordnung zur Aufnahme eines Amputationsstumpfes und einer Rahmeneinrichtung zur Verbindung der Schalenanordnung mit einer Prothese, umfasst die Schalenanordnung zumindest zwei einander gegenüberliegend angeordnete, einen Aufnahmeraum umschließende Schalenteile, die über die Rahmeneinrichtung miteinander verbunden und in ihrer Relativanordnung veränderbar sind. Die Schalenanordnung der erfindungsgemäßen Prothesenvorrichtung ist zumindest teilweise mit einer evakuierbaren, eine Vielzahl von Formpartikeln enthaltenden Auskleidung versehen und mit einer Befestigungseinrichtung zur Fixierung der Relativanordnung der Schalenteile.

Auf Grund der Kombination der in ihrem Aufnahmevolumen veränderbaren Schalenanordnung mit der evakuierbaren Auskleidung, die unabhängig vom aktuellen Stumpfvolumen eine stets formschlüssige Aufnahme des Amputationsstumpfs in der Schalenanordnung ermöglicht, kann im Zusammenwirken mit der Befestigungseinrichtung, die eine Fixierung der Schalenteile zueinander ermöglicht, der jeweils für den Heilungsvorgang förderliche Umfangsdruck ohne die Ausbildung von Druckstellen auf den Amputationsstumpf ausgeübt werden kann. Hierdurch wird in idealer Weise die Stumpfreifung unterstützt.

Darüber hinaus bietet die Schalenanordnung im Zusammenwirken mit der evakuierbaren Auskleidung aber auch die Möglichkeit, eine Kraftübertragung über im Wesentlichen die gesamte Stumpfoberfläche vom Amputationsstumpf auf eine mit der Rahmeneinrichtung verbindbare Prothese, also beispielsweise eine Gehstütze, zu schaffen.

Damit kann die erfindungsgemäße Prothesenvorrichtung in gleicher Weise vorteilhaft zur Unterstützung der Stumpfreifung, als auch zum Anschluss einer Funktionsprothese an den Amputationsstumpf genutzt werden.

Bei einer vorteilhaften Ausführungsform der Prothesenvorrichtung sind die Schalenteile so mit der Rahmeneinrichtung verbunden, dass sowohl der lichte Abstand d_{S} der Schalenteile zueinander als auch der zwischen

Längsachsen der Schalenteile gebildete Schalenöffnungswinkel α_{S} veränderbar ist. Zum einen ist hiermit in besonderem Maße eine Anpassung der Schalenkonfiguration an den Amputationsstumpf möglich. Zum anderen ist es möglich, für den Vorgang des Anlegens der Prothesenvorrichtung am Amputationsstumpf die Schalenanordnung möglichst weit zu öffnen, um den Vorgang des Anlegens zu erleichtern.

Wenn die Schalenteile formelastisch ausgebildet sind und in Längsrichtung verlaufende Schalenränder aufweisen, deren die Schalenweite definierender Abstand d_{W} veränderbar ist, kann eine Formanpassung der Schalenanordnung vorgenommen werden.

Die Formanpassung der Prothesenvorrichtung kann insbesondere dadurch erleichtert werden, dass zur Veränderung der Schalenweite d_{W} der Schalenteile die Rahmeneinrichtung zwei in ihrem Abstand d_{R} zueinander veränderbare Rahmenschenkel aufweist, die über eine gemeinsame Rahmenbasis miteinander verbunden sind und jeweils mit einem der zwei einander gegenüberliegenden Schalenränder der Schalenteile verbunden sind.

Wenn darüber hinaus die Schalenteile Langlöcher zur Verbindung mit den Rahmenschenkeln der Rahmeneinrichtung aufweisen, sind die Schalenteile auch in ihrer Positionierung gegenüber den Rahmenschenkeln veränderbar.

Bei einer bevorzugten Ausführungsform der Prothesenvorrichtung ist das auf der Streckseite benachbart dem Knie- oder Ellbogengelenk angeordnete Schalenteil (streckseitiges Schalenteil) starr mit den Rahmenschenkeln verbunden und das auf einer Beugeseite angeordnete Schalenteil (beugeseitiges Schalenteil) an den Rahmenschenkeln verschwenkbar angeordnet oder umgekehrt.

Wenn das streckseitige Schalenteil im Knie- oder Ellbogenbereich eine Ausnehmung aufweist, ist eine vereinfachte Anpassung an den Gelenkbereich möglich.

Wenn das streckseitige Schalenteil sich über den Knie- oder Ellbogenbereich hinaus erstreckt, kann das streckseitige Schalenteil auch zur Zwangsstreckung der Extremität eingesetzt werden.

Alternativ zu der vorstehenden gelenkübergreifenden Ausbildung des streckseitigen Schalenteils ist es jedoch auch möglich, dass sich das streckseitige Schalenteil mit seinem proximalen Ende bis in den Kniebereich erstreckt und sich das beugeseitige Schalenteil mit seinem proximalen Ende über den Knie- oder Ellbogenbereich hinaus erstreckt.

Vorteilhaft ist es, wenn das beugeseitige Schalenteil an seinem proximalen Ende zwei Schalenlaschen aufweist, die mit einer Befestigungseinrichtung zur Fixierung der Relativanordnung der Schalenlaschen versehen sind. Hierdurch wird ein Umschließen der Anordnung der Schalenanordnung auch oberhalb des Gelenkbereichs möglich.

Alternativ zu einer gelenkübergreifenden Ausbildung des beugeseitigen Schalenteils ist es auch möglich, dass sich das streckseitige Schalenteil mit seinem proximalen Ende bis in den Knie- oder Ellbogenbereich erstreckt und mit einem Schienenmodul versehen ist, das sich mit seinem proximalen Ende über den Knie- oder Ellbogenbereich hinaus erstreckt und an seinem proximalen Ende mit einer Befestigungseinrichtung versehen ist.

Zur gelenkübergreifenden Anpassung der Prothesenvorrichtung an die Extremität ist es vorteilhaft, wenn das Schienenmodul im Knie- oder Ellbogenbereich mit einer Gelenkeinrichtung versehen ist.

Um eine Quengelung in definierter Beugestellung der Extremität oder einen definierten Beuge- oder Bewegungsbereich zu ermöglichen, kann die Gelenkeinrichtung mit einer Einstelleinrichtung zur Einstellung eines definierten Beugewinkels und/oder eines definierten Beugebereichs versehen sein.

Nachfolgend werden bevorzugte Ausführungsformen der Prothesenvorrichtung anhand der Zeichnung näher erläutert.

Es zeigen:
- **Fig. 1:**: eine erste Ausführungsform einer Prothesenvorrichtung in Draufsicht;
- **Fig. 2:**: die in **Fig. 1** dargestellte Prothesenvorrichtung in Seitenansicht und mit geöffneter Schalenanordnung;
- **Fig. 3:**: eine Rahmeneinrichtung einer Prothesenvorrichtung mit einem angeschlossenen Stützteil;
- **Fig. 4:**: eine zweite Ausführungsform einer Prothesenvorrichtung in Draufsicht;
- **Fig. 5:**: die in **Fig. 4** dargestellte Prothesenvorrichtung in Seitenansicht und mit geöffneter Schalenanordnung;
- **Fig. 6:**: eine dritte Ausführungsform einer Prothesenvorrichtung in Draufsicht;
- **Fig. 7:**: die in **Fig. 6** dargestellte Prothesenvorrichtung in Seitenansicht und mit geöffneter Schalenanordnung.

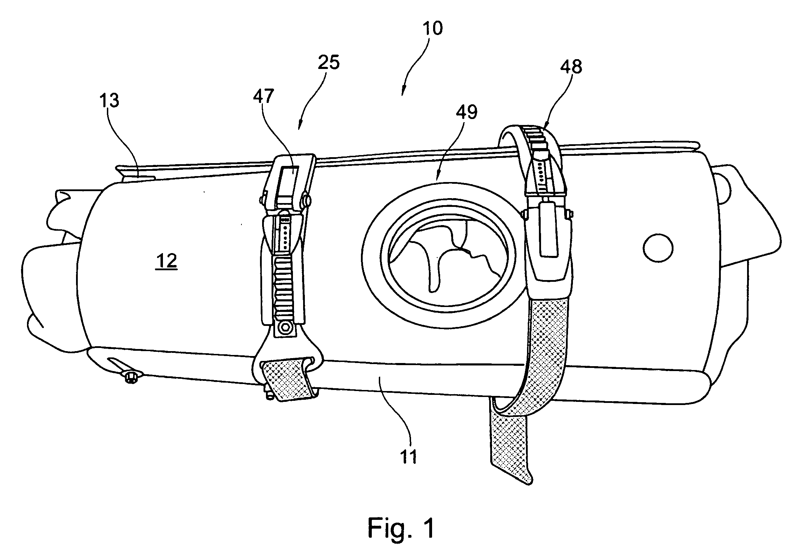

**Fig. 1** zeigt in einer ersten Ausführungsform eine Prothesenvorrichtung 10 mit einer Wadenschale 11 und einer Schienbeinschale 12 in geschlossenem Zustand, in dem die Wadenschale 11 und die Schienbeinschale 12 über eine durch zwei Zugbänder 47, 48 gebildete Befestigungseinrichtung 25 gegeneinander fixiert und verbunden sind. Die Schienbeinschale 12 weist eine Kniescheibenöffnung 49 zur Aufnahme einer hier nicht näher dargestellten Kniescheibe bei einem in die Prothesenvorrichtung 10 eingesetzten, am Unterschenkel ausgebildeten Amputationsstumpf au f.

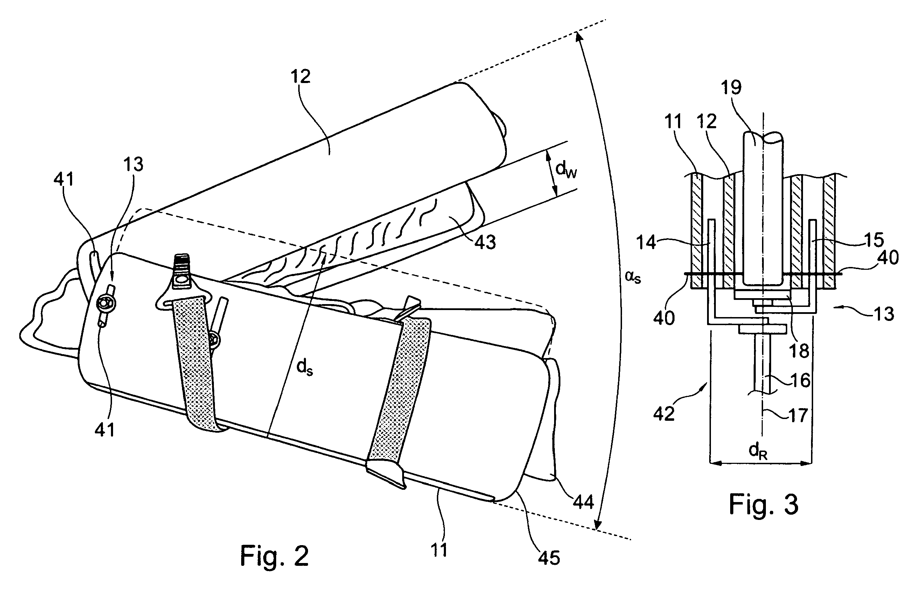

Wie aus der in **Fig. 2** dargestellten geöffneten oder Einstiegs-Stellung der Prothesenvorrichtung 10 deutlich wird, sind die Wadenschale 11 und die Schienbeinschale 12 an ihrem distalen Ende mit einer Rahmeneinrichtung 13 verbunden, die in **Fig. 3** in Seitenansicht dargestellt ist. Die Rahmeneinrichtung 13 weist zwei hier L-förmig ausgebildete Rahmenschenkel 14, 15 auf, die im Bereich ihrer Schenkelbasis 42 an eine Prothese, von der in **Fig. 3** lediglich ein Stützteil 16 dargestellt ist, angeschlossen sind. Wie aus **Fig. 3** zu ersehen ist, sind die beiden Rahmenschenkel 14 und 15 radial gegeneinander, also relativ zu einer Hochachse 17, verschiebbar. Bei dem vorliegenden Ausführungsbeispiel bilden die einander überlappenden Teile der Rahmenschenkel 14, 15 eine Basis 42, an die das Stützteil 16 angeschlossen ist. Die Rahmenschenkel 14, 15 liegen mit ihren Innenseiten an der Außenwandung der Schienbeinschale 12 und mit ihren Außenseiten an der Innenwand der Wadenschale 11 an und sind mit diesen über Bolzenverbindungen 40 gegeneinander verschwenkbar bzw. mittels in **Fig. 2** dargestellter Langlöcher 41 gegeneinander verschiebbar befestigt. Auf der Basis 42 liegt eine Polsterung 18 zur zwischenliegenden Anordnung zwischen der Basis 42 und einem Amputationsstumpf 19 auf.

Abweichend von der Darstellung in **Fig. 3** können die Rahmenschenkel 14, 15 der Rahmeneinrichtung 13 gegenüber ihrer Basis 42, die zum Anschluss einer Prothese 16 dient, um eine Hochachse 17 der Prothese abwinkelbar ausgeführt sein. Hierdurch lässt sich in vorteilhafter Weise eine Einstellung des Körperschwerpunkts bzw. des sich beim Gehen einstellenden dynamischen Schwerpunkts relativ zur Prothesenauftrittsfläche einstellen, so dass beispielsweise unnötige Stumpfbelastungen durch auftretende Querkräfte möglichst gering gehalten werden können.

Wie insbesondere aus **Fig. 2** zu ersehen, ist jeweils in der Schienbeinschale 12 und in der Wadenschale 11 ein Vakuumkissen 43 bzw. 44 angeordnet, das mit einer gasdichten Außenhülle 45 und einer Vielzahl von darin angeordneten, hier nicht näher dargestellten Formpartikeln versehen ist. Weiterhin weisen die Vakuumkissen 43, 44, hier nicht näher dargestellte Ventileinrichtungen auf, über die eine Evakuierung der Vakuumkissen 43, 44 durchführbar ist.

In der in **Fig. 2** dargestellten geöffneten Konfiguration ist die Prothesenvorrichtung 10 für den Einstieg, also das Einlegen des Amputationsstumpfs 19, vorbereitet. Nach dem Einlegen werden die Wadenschale 11 und die Schienbeinschale 12 gegeneinander zur formschlüssigen Anlage an den Stumpf 19 verschwenkt und mit der Befestigungseinrichtung 25 in ihrer Relativanordnung fixiert. Anschließend erfolgt durch Evakuierung der Vakuumkissen 43, 44 eine formschlüssige Anpassung an die Kontur des Amputationsstumpfs 19, so dass die Formkörperfüllung der Vakuumkissen 43, 44 stützend angeschmiegt am Amputationsstumpf 19 anliegt. Über die formschlüssige Anpassung wird zum einen durch eine Kompressionswirkung auf den Stumpf die Stumpfreifung unterstützt und zum anderen ermöglicht die formschlüssige Anpassung, die aufgrund der in **Fig. 1** dargestellten Kniescheibenöffnung 49 auch einen Formschluss im Kniebereich ermöglicht, eine Abstützung in Belastungsrichtung, so dass Reaktionskräfte beim Gehen durch die Prothesenvorrichtung 10 im Bereich der Vakuumkissen 43, 44 flächig aufgenommen werden.

Wie eine Zusammenschau der **Fig. 2** und **3** zeigt, sind die Schalenteile 11, 12 so mit der Rahmeneinrichtung 13 verbunden, dass sowohl der lichte Abstand d_{S} als auch der Schalenöffnungswinkel α_{S} veränderbar ist. Zur Veränderung der Schalenweite d_{W} der Schalenteile 11, 12 sind die zwei Rahmenschenkel 14, 15 in ihrem Abstand d_{R} zueinander veränderbar und jeweils mit Schalenrändern 55, 56 verbunden.

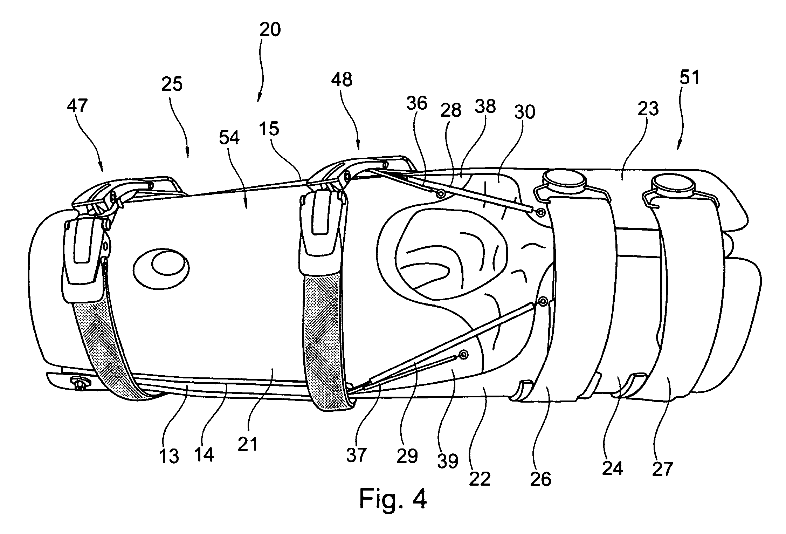

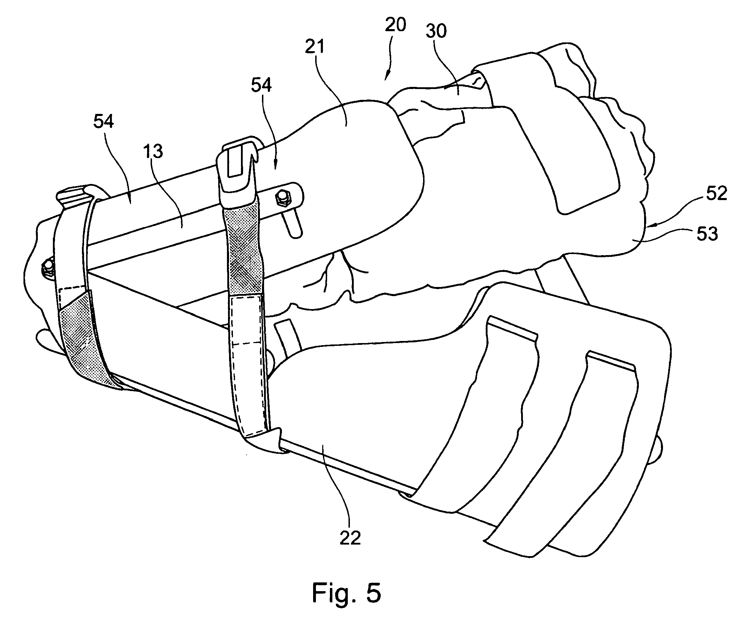

In den **Fig. 4** und **5** ist eine Prothesenvorrichtung 20 dargestellt, die eine gegenüber einer Schienbeinschale 21 verlängerte Wadenschale 22 aufweist. Die Wadenschale 22 ist so aufgebaut, dass sie oberhalb eines in der Schienbeinschale 21 ausgebildeten Kniebereichs 50 sich aufeinander zu erstreckende Oberschenkellaschen 23, 24 aufweist, die ergänzend zu der Befestigungseinrichtung 25, deren beide Zugbänder 47, 48 die Fixierung der Schalenteile 21 und 22 im Stumpfbereich übernehmen, eine hier aus zwei Gurtbändern 26, 27 gebildete proximale Befestigungseinrichtung 51 aufweisen, die für einen Kraftschluss der Oberschenkellaschen 23, 24 oberhalb des Knies sorgt. Wie in **Fig. 4** angedeutet, können die Rahmenschenkel 14, 15 der Rahmeneinrichtung 13 über Druckstäbe 28, 29 mit den Oberschenkellaschen 23, 24 verbunden sein, um das Anliegen der Oberschenkellaschen 23, 24 am Oberschenkel oberhalb des Knies zu unterstützen. Alternativ oder zusätzlich können Druckstäbe 36, 37 vorgesehen sein, die auf den Kniebereich 50 definierende Knielaschen 38, 39 der Schienbeinschale 21 wirken, um unterhalb des Knies eine den Formschluss unterstützende Anschmiegung der Knielaschen 38, 39 zu bewirken.

Wie aus Fig. 5 hervorgeht, ist ein in der Prothesenvorrichtung 20 vorgesehenes Vakuumkissen 30 einteilig ausgebildet und derart angeordnet, dass freie, einander gegenüberliegende Kissenränder 52, 53 im Wadenbereich zu liegen kommen.

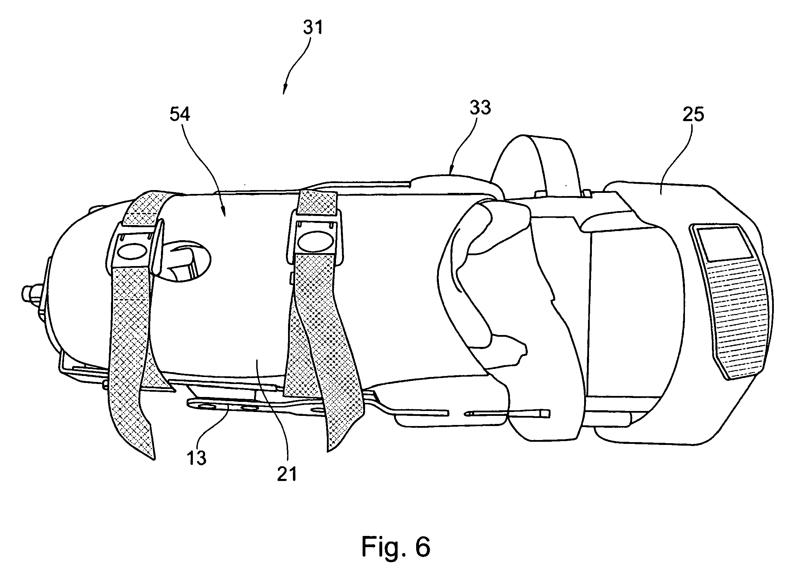

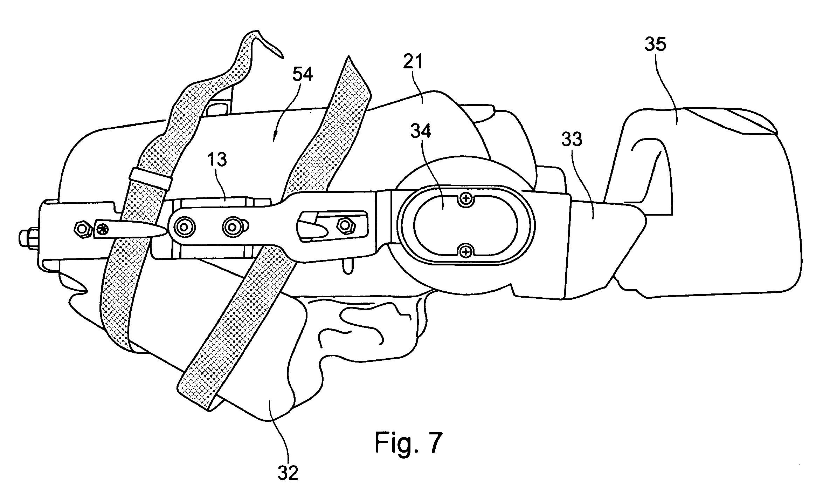

In den **Fig. 6** und **7** ist gemäß einer weiteren Ausführungsform eine Prothesenvorrichtung 31 dargestellt, die übereinstimmend mit der in den **Fig. 4** und **5** dargestellten Prothesenvorrichtung 20 mit einer Schienbeinschale 21 versehen ist. Im Unterschied zu der Prothesenvorrichtung 20 weist die Prothesenvorrichtung 31 eine relativ kurze Wadenschale 32 (**Fig. 7**) auf. Statt dessen ist jedoch die Schienbeinschale 21 vermittels der Rahmeneinrichtung 13 mit einem Schienenmodul 33 versehen, das mit einer feststellbaren Gelenkeinrichtung 34 und einem Oberschenkelhalter 35 zur Fixierung am Oberschenkel versehen ist. Das Schienenmodul 33 kann als Quengelschiene, also zur Erzielung einer Zwangsstreckung, in ähnlicher Weise wie die Wadenschale 22 der in den **Fig. 4** und 5 dargestellten Prothesenvorrichtung 20 verwendet werden. Aufgrund der Gelenkeinrichtung 34 können jedoch auch definierte Zwangsbeugewinkel oder auch gegebenenfalls Beugebereiche, in denen ein Verschwenken in einem festgelegten Beugungswinkelbereich möglich ist, definiert werden.

Bei den Prothesenvorrichtungen 20 und 31 kann jeweils eine aus der Rahmeneinrichtung 13 und der Schienbeinschale 21 gebildete Montagegruppe als Basiseinheit 54 angesehen werden, die modular, also beispielsweise durch die Wadenschale 22 oder die Wadenschale 32 und das Schienenmodul 33, ergänzt werden kann.

## Patentansprüche

1. Prothesenvorrichtung (10, 20, 31) mit einer Schalenanordnung zur Aufnahme eines Amputationsstumpfes und einer Rahmeneinrichtung (13) zur Verbindung der Schalenanordnung mit einer Prothese (16), wobei die Schalenanordnung zumindest zwei einander gegenüberliegend angeordnete, einen Aufnahmeraum umschließende Schalenteile (11, 12; 21, 22; 32) umfasst, und die Schalenanordnung mit einer Befestigungseinrichtung (25, 51) zur Fixierung der Relativanordnung der Schalenteile versehen ist,
**dadurch gekennzeichnet, dass** die Schalenteile (11, 12, 21, 22, 32) über die Rahmeneinrichtung miteinander verbunden und ihrer Relativanordnung veränderbar sind, und die Schalenanordnung zumindest teilweise mit einer evakuierbaren, eine Vielzahl von Formpartikeln (46) enthaltenen Auskleidung (45) versehen ist.

2. Prothesenvorrichtung nach Anspruch 1,
**dadurch gekennzeichnet,**
**dass** die Schalenteile (11, 12; 21, 22; 32) so mit der Rahmeneinrichtung (13) verbunden sind, dass sowohl der lichte Abstand d_{S} der Schalenteile zueinander als auch der zwischen Längsachsen der Schalenteile gebildete Schalenöffnungswinkel α_{S} veränderbar ist.

3. Prothesenvorrichtung nach Anspruch 1 oder 2,
**dadurch gekennzeichnet,**
**dass** die Schalenteile (11, 12; 21, 22; 32) formelastisch ausgebildet sind und in Längsrichtung verlaufende Schalenränder aufweisen, deren die Schalenweite definierender lichter Abstand veränderbar ist.

4. Prothesenvorrichtung nach Anspruch 3,
**dadurch gekennzeichnet,**
**dass** zur Veränderung der Schalenweite der Schalenteile die Rahmeneinrichtung (13) zwei in ihrem Abstand d_{R} zueinander veränderbare Rahmenschenkel (14, 15) aufweist, die über eine gemeinsame Rahmenbasis (42) miteinander verbunden sind und jeweils mit einem von zwei einander gegenüberliegenden Längsrändern der Schalenteile verbunden sind.

5. Prothesenvorrichtung nach einem der vorangehenden Ansprüche,
**dadurch gekennzeichnet,**
**dass** die Schalenteile (11, 12; 21, 22; 32) Langlöcher (41) zur Verbindung mit den Rahmenschenkeln (14, 15) der Rahmeneinrichtung (13) aufweisen.

6. Prothesenvorrichtung nach Anspruch 5,
**dadurch gekennzeichnet,**
**dass** das auf einer Streckseite des benachbart des Knie- oder Ellbogengelenks ausgebildeten Amputationsstumpfes angeordnete Schalenteil (streckseitige Schalenteil) (12, 21) starr mit den Rahmenschenkeln (14, 15) verbunden ist und das auf einer Beugeseite angeordnete Schalenteil (beugeseitige Schalenteil) (11, 22) an den Rahmenschenkeln (14, 15) verschwenkbar angeordnet ist oder umgekehrt.

7. Prothesenvorrichtung nach einem der vorangehenden Ansprüche,
**dadurch gekennzeichnet,**
**dass** das streckseitige Schalenteil (12, 21) in einem Knie- oder Ellbogenbereich eine Ausnehmung aufweist.

8. Prothesenvorrichtung nach Anspruch 7,
**dadurch gekennzeichnet,**
**dass** das streckseitige Schalenteil (12, 21) sich über den Knie- oder Ellbogenbereich hinaus erstreckt.

9. Prothesenvorrichtung nach einem der Ansprüche 1 bis 7,
**dadurch gekennzeichnet,**
**dass** sich das streckseitige Schalenteil (12, 21) mit seinem proximalen Ende bis in den Kniebereich und sich das beugeseitige Schalenteil (11, 22) mit seinem proximalen Ende über dem Knie- oder Ellbogenbereich hinaus erstreckt.

10. Prothesenvorrichtung nach Anspruch 9,
**dadurch gekennzeichnet,**
**dass** das beugeseitige Schalenteil (11, 22) an seinem proximalen Ende zwei die Schalenteilöffnung reduzierende Schalenlaschen 23, 24 aufweist, die mit einer weiteren Befestigungseinrichtung (51) zur Fixierung der Relativanordnung der Schalenlaschen versehen sind.

11. Prothesenvorrichtung nach einem der Ansprüche 1 bis 7,
**dadurch gekennzeichnet,**
**dass** sich das streckseitige Schalenteil (12, 21) mit seinem proximalen Ende bis in den Knie- oder Ellbogenbereich erstreckt und mit einem Schienenmodul (33) versehen ist, das sich mit seinem proximalen Ende über den Knie- oder Ellbogenbereich hinaus erstreckt und an seinem proximalen Ende mit einer Befestigungseinrichtung versehen ist.

12. Protheseneinrichtung nach Anspruch 11,
**dadurch gekennzeichnet,**
**dass** das Schienenmodul (33) im Knie- oder Ellbogenbereich mit einer Gelenkeinrichtung (34) versehen ist.

13. Protheseneinrichtung nach Anspruch 12,
**dadurch gekennzeichnet,**
**dass** die Gelenkeinrichtung (34) mit einer Einstelleinrichtung zur Einstellung eines definierten Beugewinkels und/oder eines definierten Beugebereichs versehen ist.

## Claims

1. A prosthetic device (10, 20, 31) having a shell arrangement for receiving an amputation stump and a frame mechanism (13) for connecting the shell arrangement to a prosthesis (16), whereby the shell arrangement comprises at least two shell parts (11, 12; 21, 22; 32) that are arranged opposite one another and surround a receptacle space, and the shell arrangement is provided with a fastening mechanism (25, 51) for securing the relative arrangement of the shell parts,
**characterized in that**
the shell parts (11, 12, 21, 22, 32) are connected to one another via the frame mechanism and are variable in their relative arrangement, and the shell arrangement is provided at least partially with an evacuable lining (45) that contains a plurality of form particles (46).

2. The prosthetic device according to Claim 1,
**characterized in that**
the shell parts (11, 12; 21, 22; 32) are connected to the frame mechanism (13) in such a way that both the inside distance d_{S} of the shell parts is variable in relation to one another and the shell opening angle α_{S} formed between the longitudinal axes of the shell parts is variable.

3. The prosthetic device according to Claim 1 or 2,
**characterized in that**
the shell parts (11, 12; 21, 22; 32) are designed with shape elasticity and have shell edges running in the longitudinal direction, the inside spacing of which defining the shell width is variable.

4. The prosthetic device according to Claim 3,
**characterized in that**
to vary the shell width of the shell parts, the frame mechanism (13) has two frame legs (14, 15) that are variable in their distance d_{R} in relation to one another and are joined together by a common frame base (42), and each is connected to one of two opposing longitudinal edges of the shell parts.

5. The prosthetic device according to any one of the preceding claims,
**characterized in that**
the shell parts (11, 12,; 21, 22; 32) have elongated holes (41) for connecting to the frame legs (14, 15) of the frame mechanism (13).

6. The prosthetic device according to Claim 5,
**characterized in that**
the shell part arranged on the extensor side of the amputation stump formed next to the knee joint or the elbow joint (extensor-sided shell part) (12, 21) is rigidly connected to the frame legs (14, 15) and the shell part (flexor-sided shell part) (11, 22) arranged on a flexor side is pivotably arranged on the frame legs (14, 15) or vice versa.

7. The prosthetic device according to any one of the preceding claims,
**characterized in that**
the extensor-sided shell part (12, 21) has a recess in a knee area or an elbow area.

8. The prosthetic device according to Claim 7,
**characterized in that**
the extensor-sided shell part (12, 21) extends beyond the area of the knee or elbow.

9. The prosthetic device according to any one of Claims 1 through 7,
**characterized in that**
the extensor-sided shell part (12, 21) extends at its proximal end into the knee area, and the flexor-sided shell part (11, 22) extends with its proximal end beyond the area of the knee or elbow.

10. The prosthetic device according to Claim 9,
**characterized in that**
the flexor-sided shell part (11, 22) has two shell straps (23, 24) at its proximal end, said shell straps reducing the size of the shell part opening and being provided with another fastening mechanism (51) for securing the relative arrangement of the shell straps.

11. The prosthetic device according to any one of Claims 1 through 7,
**characterized in that**
the extensor-side shell part (12, 21) extends with its proximal end into the area of the knee or elbow and is provided with a rail module (23) which extends at its proximal end beyond the area of the knee or elbow and is provided with a fastening mechanism at its proximal end.

12. The prosthetic device according to Claim 11,
**characterized in that**
the shell module (33) is provided with an articular mechanism (34) in the area of the knee or elbow.

13. The prosthetic device according to Claim 12,
**characterized in that**
the articular mechanism (34) is provided with an adjustment mechanism for adjusting a defined flexor angle and/or defined range of flexion.

## Revendications

1. Dispositif de prothèse (10, 20, 31) comportant un système de coques destiné à recevoir un moignon d'amputation et un système d'armature (13) afin de relier le système de coques à une prothèse (16), le système de coques comprenant au moins deux parties de coque (11, 12; 21, 22; 32) définissant un espace de réception, disposées en vis-à-vis l'une de l'autre, et le système de coques étant pourvu d'un dispositif de fixation (25, 51) pour fixer la disposition relative des parties de coque,
**caractérisé en ce que**
les parties de coque (11, 12; 21, 22; 32) sont reliées les unes aux autres par le système d'armature et leur disposition relative peut être modifiée, et le système de coques est pourvu au moins partiellement d'un revêtement (45) pouvant être évacué et contenant une pluralité de particules à mouler (46).

2. Dispositif de prothèse selon la revendication 1,
**caractérisé en ce que**
les parties de coque (11, 12, 21, 22, 32) sont reliées au système d'armature (13) de telle sorte que tant la tolérance dₛ entre les parties de coque que l'angle d'ouverture de coque αₛ formé entre les axes longitudinaux des parties de coque puissent être modifiés.

3. Dispositif de prothèse selon la revendication 1 ou 2,
**caractérisé en ce que**
les parties de coque (11, 12; 21, 22; 32) ont une configuration élastiquement déformable et présentent des bords de coques s'étendant dans la direction longitudinale, dont la tolérance définissant la largeur de coque peut être modifiée.

4. Dispositif de prothèse selon la revendication 3,
**caractérisé en ce que**
afin de modifier la largeur de coque des parties de coque, le système d'armature (13) présente deux pans d'armature (14, 15) dont l'espacement d_{R} l'un par rapport à l'autre peut être modifié, qui sont reliés l'un à l'autre par l'intermédiaire d'une base d'armature (42) commune et qui sont respectivement reliés à un des deux bords longitudinaux des parties de coque se faisant face.

5. Dispositif de prothèse selon une des revendications précédentes,
**caractérisé en ce que**
les parties de coque (11, 12; 21, 22; 32) présentent des alésages oblongs (41) pour la liaison avec les pans d'armature (14,15) du système d'armature (13).

6. Dispositif de prothèse selon la revendication 5,
**caractérisé en ce que**
la partie de coque disposée sur un côté d'étirement du moignon d'amputation situé à côté de l'articulation du genou ou du coude (partie de coque du côté d'étirement) (12, 21) est reliée rigidement aux pans d'armature (14, 15) et la partie de coque disposée sur un côté de flexion (partie de coque du côté de flexion) (11, 22) est disposée de manière basculable sur les pans d'armature (14, 15) ou inversement.

7. Dispositif de prothèse selon une des revendications précédentes,
**caractérisé en ce que**
la partie de coque du côté d'étirement (12, 21) présente une cavité dans la région du genou ou du coude.

8. Dispositif de prothèse selon la revendication 7,
**caractérisé en ce que**
la partie de coque du côté d'étirement (12, 21) s'étend par-dessus la région du genou ou du coude.

9. Dispositif de prothèse selon une des revendications 1 à 7,
**caractérisé en ce que**
la partie de coque du côté d'étirement (12, 21) s'étend par son extrémité proximale jusqu'à la région du genou et la partie de coque du côté de flexion (11, 22) s'étend par son extrémité proximale par-dessus la région du genou ou du coude.

10. Dispositif de prothèse selon la revendication 9,
**caractérisé en ce que**
la partie de coque du côté de flexion (11, 22) présente à son extrémité proximale deux pattes de fixation de coque (23, 24) réduisant l'ouverture de la partie de coque et qui sont pourvues d'un dispositif de fixation (51) supplémentaire afin de fixer la disposition relative des pattes de fixation de coque.

11. Dispositif de prothèse selon une des revendications 1 à 7,
**caractérisé en ce que**
la partie de coque du côté d'étirement (12, 21) s'étend par son extrémité proximale jusqu'à la région du genou ou du coude et est pourvue d'un module de rail (33), qui s'étend par son extrémité proximale par-dessus la région du genou ou du coude et est pourvu à son extrémité proximale d'un dispositif de fixation.

12. Dispositif de prothèse selon la revendication 11,
**caractérisé en ce qu'**
le module de rail (33) est pourvu dans la région du genou ou du coude d'un dispositif de joint articulé (34).

13. Dispositif de prothèse selon la revendication 12,
**caractérisé en ce que**
le dispositif de joint articulé (34) est pourvu d'un dispositif de réglage pour régler un angle de flexion défini et/ou une zone de flexion définie.
